# EUROPEAN PATENT SPECIFICATION

(11) **EP 2 675 888 B1**
(45) Date of publication and mention of the grant of the patent: **05.04.2017**
(21) Application number: 12702855.3
(22) Date of filing: 10.02.2012
(51) Int. Cl.: C12M 1/34, G01N 27/403, G01N 33/487, C40B 30/06

(54) **NEURONAL NETWORK BASED BIOSENSOR**
AUF NEURONALEM NETZWERK BASIERENDER BIOSENSOR
BIOCAPTEUR À BASE DE RÉSEAU NEURONAL

(30) Priority: 17.02.2011 EP 11154830
(43) Date of publication of application: 25.12.2013
(73) Proprietor: The European Union, represented by the European Commission, 1049 Brussels (BE)
(72) Inventor: SOBANSKI, Tomasz, 00120 Helsinki (FI); NOVELLINO, Antonio, 16148 Genova (GE) (IT); WHELAN, Maurice, 21021 Angera (IT)
(74) Representative: Office Freylinger
(86) International application number: PCT/EP2012/052346
(87) International publication number: WO 2012/110426

(56) References cited:
- US-A1- 2009 322 309
- BUZANSKA L ET AL: "Neural stem cells from human cord blood on bioengineered surfacesNovel approach to multiparameter bio-tests", TOXICOLOGY, LIMERICK, IR, vol. 270, no. 1, 30 March 2010 (2010-03-30), pages 35-42, XP026982364, ISSN: 0300-483X [retrieved on 2009-06-16]
- BERDONDINI L ET AL: "A microelectrode array (MEA) integrated with clustering structures for investigating in vitro neurodynamics in confined interconnected sub-populations of neurons", SENSORS AND ACTUATORS B, ELSEVIER SEQUOIA S.A., LAUSANNE, CH, vol. 114, no. 1, 30 March 2006 (2006-03-30), pages 530-541, XP025112077, ISSN: 0925-4005, DOI: DOI:10.1016/J.SNB.2005.04.042 [retrieved on 2006-03-30]
- MELANIE JUNGBLUT ET AL: "Triangular neuronal networks on microelectrode arrays: an approach to improve the properties of low-density networks for extracellular recording", BIOMEDICAL MICRODEVICES, KLUWER ACADEMIC PUBLISHERS, BO, vol. 11, no. 6, 15 September 2009 (2009-09-15), pages 1269-1278, XP019746536, ISSN: 1572-8781, DOI: DOI:10.1007/S10544-009-9346-0
- JUN S B ET AL: "Low-density neuronal networks cultured using patterned poly-l-lysine on microelectrode arrays", JOURNAL OF NEUROSCIENCE METHODS, ELSEVIER SCIENCE PUBLISHER B.V., AMSTERDAM, NL, vol. 160, no. 2, 15 March 2007 (2007-03-15), pages 317-326, XP026863301, ISSN: 0165-0270 [retrieved on 2007-02-15]
- IAN L JONES ET AL: "The potential of microelectrode arrays and microelectronics for biomedical research and diagnostics", ANALYTICAL AND BIOANALYTICAL CHEMISTRY, SPRINGER, BERLIN, DE, vol. 399, no. 7, 31 July 2010 (2010-07-31) , pages 2313-2329, XP019880474, ISSN: 1618-2650, DOI: DOI:10.1007/S00216-010-3968-1

## Description

### FIELD OF THE INVENTION

The present invention generally relates to the field of neuronal network based biosensors and more specifically to a biosensor structure with micro-electrode array for cultured neurons.

### BACKGROUND OF THE INVENTION

Cultured neuronal networks have the capacity to respond to a wide range of neuroactive compounds and are conventionally used in biosensing applications, e.g. for screening known and unknown compounds for neuro-active effects.

Applications of this *in vitro* approach range from the ability to screen potential drugs for deleterious neuropharmacologic side effects to the detection of neurotoxins and neurotoxicants. The capacity to interpret the effects of unknown compounds requires the monitoring of physiologically relevant activity in these cultured cells systems.

In this context, micro-electrode arrays (MEAs) are now conventionally employed as a tool for monitoring and/or electrically stimulating cultured neurons, and are preferred to the so-called patch clamp technique.

In the manufacture of a typical MEA biosensor, the MEA surface is prepared and coated with an adhesion-promoting layer (e.g. Poly-D-Lysine or Poly-L-Lysine and laminin). Neurons are then seeded on the coated MEA and cultured under controlled conditions. Hence, a conventional MEA biosensor comprises a random monolayer of cultured neurons thereon.

A spatially organized growth of the neuronal network is however desirable to improve the biochemical connection between the neurons and the electrode surface, which is required to overcome problems such as low electrical signals and precise positioning of the neuronal cells onto the desired locations on the MEA surface.

This is why a variety of surface patterning techniques have been developed in order to control the growth of neurons into networks, and thus provide a controlled network topology. These include: micro-contact printing, photolithography, soft lithography, micro-stamping and ink-jet printing.

The *in vitro* study of neuronal networks on MEAs is e.g. described by L. Berdondini et al. in "A microelectrode array (MEA) integrated with clustering structures for investigating in vitro neurodynamics in confined interconnected sub-populations of neurons", Sensors and Actuators B 114 (2006), 530-541, Elsevier.

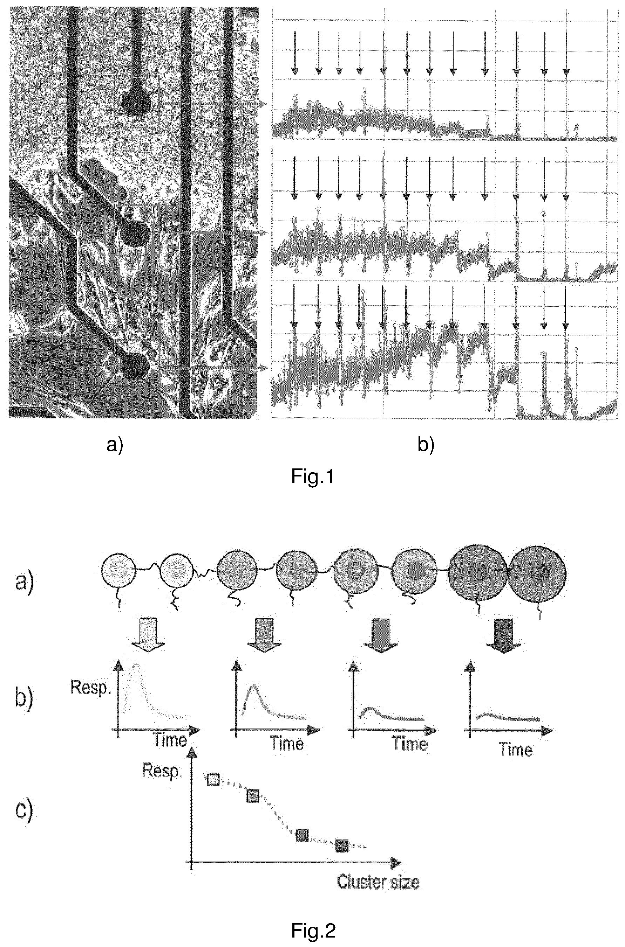

This document describes a biosensor substrate comprising a microelectrode array (MEA) and a pattern of clusters of two sizes (see figure 1, clusters B-D on the one hand and cluster A on the other hand) coated with neuronal cells.

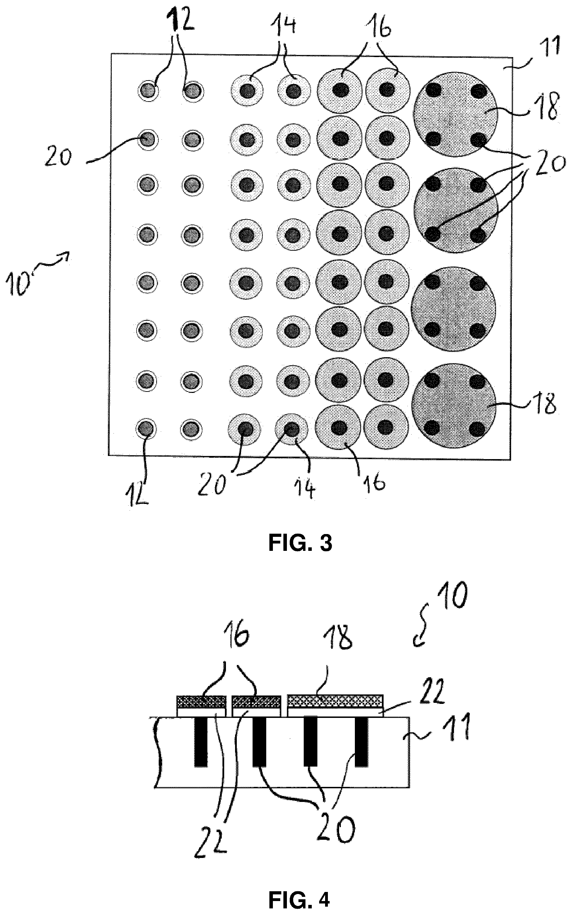

Micro-technologies to create cell patterns on a substrate are described by L. Buzanska et al. in "Neural stem cells from human cord blood on bioengineered surfaces - Novel approach to multiparameter biotests", Toxicology 270 (2010) 35-42, Elsevier Ireland. An example of the possibilities offered by micro-patterning is shown in Fig.4, where a fibronectin array layout with increasing drop size and protein concentration has been formed on plain substrate, without electrode means. This layout was designed to test and optimize the spotting density and the spot size most suitable for differentiation process of HUCB-NSC stem cells.

### OBJECT OF THE INVENTION

The objective of the present invention is to provide a biosensor structure that allows for a higher sensitivity of the neuronal network to exogenous application of tests compounds and better control of the network response.

### SUMMARY OF THE INVENTION

This objective is achieved by a biosensor substrate as claimed in claim 1. Preferred embodiments are recited in dependent claims 2 to 4.

The proposed patterned structure indeed provides a guiding substrate for the growth of cultured cells in a predetermined topologically organised manner, by means of cytophilic pads of defined size and position (spatial location) on the substrate. The cytophilic pads are islands of material that retain cells and promote their growth, i.e. material adapted for hosting and culturing cells deposited thereon. The present approach allows a higher sensitivity of the neuronal network to exogenous application of tests compounds and better spatial control of the network response.

As a matter of fact, in developing this invention, the present inventors have observed that, during measurement of neuronal activity on MEA chips, the network structure-and in particular the cluster size-affects quantitatively and qualitatively the obtained results. The parameters affected significantly are: the number of active electrodes, the shape and the frequency of the spikes, the burst frequency and also the electrical activity patterns.

Additionally, the inventors have observed that the neuronal network sensitivity and reproducibility are linked to the neuronal network structure, particularly to the number of clusters and their size. It appears that the functional properties of the neuronal tissue are strictly related to the network structure (number of cells interacting together) therefore parameters like network complexity (related to cluster size) will strongly influence these parameters. Moreover, bigger clusters can form structures similar to 3D aggregates where the penetration of the chemical agent can be less efficient as compared to small aggregates grown as monolayer. Consequently, the inventors have concluded that bigger clusters are less sensitive than smaller ones.

As a result, the present invention also concerns a biosensor comprising cultured cells, namely in the form of neuron clusters, arranged on a substrate with electrode means for monitoring and/or stimulating the electrical activity of the neuronal cells.

The substrate has a structured surface, on which the cells are arranged according to a predetermined pattern in spatially organised clusters of controlled size. The clusters exhibit a size distribution comprising a predetermined number of distinct size classes that is not less than four, each size class corresponding to a given range of cluster area; and the cluster area between two consecutive size classes is increased by a factor of four or more.

The term cluster herein designates an aggregate of cells, typically in 3D and thus opposes to a monolayer structure. It is considered that a minimum cluster size is an aggregate of between 3 to 10 close cells, namely 3 to 10 close neurons.

Hence, the present invention provides a biosensor allowing for a predetermined arrangement of clusters of controlled size and position. Such biosensors with a controlled structure, allow a higher degree of reproducibility between similar experiments as well as an enhanced sensitivity and extended working range. Indeed, controlling the cluster spatial distribution allows assigning given electrodes with clusters of defined and reproducible size, which reduces the variations in electric activity patterns. The enhanced sensitivity arises from the use of clusters of different, known size (pre-determinable size) and from higher signal-to-noise ratio due to the lower variability of electric activity. In fact clusters of different size respond differently to the application of the same compound and the sensitivity depends on the size of the cluster itself.

In practice, these results provide an extended working range for the biosensor.

Another benefit of the present biosensor is the simplification of signal processing. At present, the random growth of cultured neurons on the biosensor implies substantial variability of the recorded signal both in terms of amplitude and intensity thus sophisticated algorithms are used to select active electrodes and normalise the signals; and this is often manually performed. The present biosensor provides a simplified network structure where signals from each cluster class can be readily processed.

It is further to be noted that controlling cluster size and spatial distribution allows designing less complex neural structures, which are easier to study and model.

The present biosensor is particularly useful for neurotoxicty testing and drug screening, but it can be used for a variety of physiological studies on neuronal cultures. While the present invention has been particularly developed for neuron cluster cultures, the present biosensor substrate may also be used with other kinds of cells, e.g. cardiac cells.

Depending on the targeted mechanism of neurotoxicity the clusters can be independent (i.e. not inter-connected) or connected with others (inter cluster connections). However, within the clusters, the cells are connected in all cases. Additionally the intra cluster connectivity level is always higher than inter cluster connections, as it will be understood by those skilled in the art.

In practice, the design of the cluster pattern (in terms of size and position) shall advantageously take into account the size and the spacing of the printed electrodes on the available biosensor (e.g. the MEA chip). In one variant, the cluster size distribution is adapted for 30 µm diameter electrodes with an electrode spacing of about 200 µm. Four cluster size classes according to the following size ranges may thus be used, expressed in equivalent diameters: 30-40 µm; 60-80 µm; 120-160 µm; 260-430 µm.

In another embodiment, the cluster size distribution is adapted for 30 µm diameter electrodes with an electrode spacing of about 500 µm. Four cluster size classes according to the following size ranges may thus be used, expressed in equivalent diameters: 30-50 µm; 120-150 µm; 260-300 µm; 560-930 µm.

The term "equivalent diameter" is used to encompass the case of cytophilic pads, respectively clusters, of non-circular shape, but having an area corresponding to the area of circles with the cited diameters.

As it will be understood by those skilled in the art, any appropriate material may be used to form the cytophilic pads. A variety of proteins may be used, and for example, but not exclusively, Poly-D-Lysine or Poly-L-Lysine and laminin.

The substrate may generally mainly consist of rigid insulator material, e.g. glass or other appropriate material, as is known in the art. It advantageously comprises electrode means embedded in the substrate. Preferably the substrate is a microelectrode array.

These and other embodiments are recited in dependent claims 8 to 11.

According to another aspect, the present invention concerns a process for preparing a biosensor according to claim 12. Preferred embodiments are recited in dependent claims 13 and 14.

According to still another aspect, the present invention concerns a method of conducting assays according to claim 15.

### BRIEF DESCRIPTION OF THE DRAWINGS

The present invention will now be described, by way of example, with reference to the accompanying drawing, in which:
- FIG. 1:: shows a top view of a network of clusters of neural cells grown on a MEA (Fig.1 a) and illustrates the relation between neuronal cluster size and their response to a treatment, via response charts that are given for three electrode locations (Fig.1 b);
- FIG. 2:: illustrates the working principle of range extension on a biosensor with spatially organised clusters of controlled size (a); and shows corresponding response curves (b) and a cluster-size dependent curve (c);
- FIG. 3:: is a sketch showing the design principle of the present biosensor (not a scaled drawing);
- FIG. 4:: is a partial vertical section view through the sensor of Fig.3 across the cluster of the two largest sizes.

### DETAILED DESCRIPTION OF A PREFERRED EMBODIMENT

During measurement of neuronal activity on MEA chips, the present inventors have observed that the network structure-and more particularly the cluster size-affects quantitatively and qualitatively the obtained results as described in the summary.

Additionally, while measuring the response to different compounds from clusters of cultured neurons the present inventors have observed that the neuronal network sensitivity and reproducibility are linked to the neuronal network structure, particularly in terms of cluster size and number. An example of how neuronal cluster size influences the electrical activity during the measurements of dose response curve is illustrated in Fig.1.

It thus appears that the functional properties of the neuronal tissue are strongly related to the network structure (namely the number of cells interacting together); therefore parameters like network complexity (related to cluster size) will strongly influence the functional properties of the cultured cells. Moreover, bigger clusters can form structures similar to 3D aggregates where the penetration of chemical agent can be less efficient as compared to small aggregates grown as monolayer. Consequently, the inventors have concluded that bigger clusters are less sensitive than smaller ones.

One can consider a single cluster as a separate sub-system where the cells possess specific morphological and physiological characteristics. Many critical features of MEA systems can thus be improved by a proper control of the network structure.

Based on the above observations, the present inventors have designed a neuronal network biosensor comprising cultured neuronal cells arranged on a substrate with electrode means for monitoring and/or stimulating the electrical activity of said neuronal cells, such as e.g. a MEA chip, where surface engineering techniques are applied to control the cell organisation and thus the growth of the neural cells according to a predetermined pattern in spatially organised clusters of controlled size.

The design of the sensor has been made to provide an enhanced working range, relying on the principle that neurons grouped in small clusters are more sensitive to the same test compound/sample since they are more exposed, as compared to neurons forming large clusters.

Fig.2 illustrates the working principle of such range extension. Clusters of controlled size are grown on the MEA chip (Fig.2 a). The response curves in line b) show how the cluster size affects the response time of clusters of varying size when tested with the same analyte concentration. These response curves can be used to build a cluster-size dependent response curve (Fig.2 c).

In the present biosensor, the clusters fall under a size distribution system comprising a predetermined number of distinct (non-overlapping) size classes that is not less than four, each size class corresponding to a given range of cluster area; and the cluster area between two consecutive size classes is increased by a factor of four or more.

As a result, the number and size of the clusters are designed to optimize the network response to chemical application.

A principle sketch of such sensor 10 is shown in Figs.3 and 4. There are four distinct, non-overlapping classes of clusters 12, 14, 16 and 18 that are spatially arranged on the sensor surface, on the top of a sensor substrate 11, and all clusters belong to one and only one of said four classes: hence there is a predetermined number of distinct classes. The sensor 10 hence has 16 clusters in the 1^{st}, 2^{nd} and 3^{rd} class, and 4 clusters in the fourth, larger class. Reference sign 20 indicates the biosensor electrodes (typically part of the MEA embedded in the substrate) that allow monitoring and/or stimulating the clusters. Here the electrodes are arranged with an identical, regular spacing. The electrodes 20 are embedded in the substrate (generally made from rigid insulating material, e.g. glass) but have one end arriving at the upper surface of the substrate 11 to establish contact with material deposited on the latter. In the present variant, clusters of the first three size classes 12, 14, 16 are each in connection with one respective electrode 20. While four electrodes 20 are preferably used to record at best the clusters 18 of the 4^{th} class.

As shown above, such a biosensor design provides an extended working range and other advantages as compared to randomly grown neural networks. The spatial distribution of the clusters is known as well as their responsiveness/sensitivity. It thus allows for an improved, more reliable and accurate data processing in use.

The manufacture of such biosensor requires a proper structure of the biosensor surface. Preferably, the controlled growth of the neural cells is obtained by coating the culture surface of the biosensor with cytophilic material that promotes the adhesion of neural cells. This coating is however done in accordance with the desired pattern of clusters: cytophilic material is deposited on the substrate surface at desired locations, where the growth of cluster is desired. This structure is best seen in Fig.4, where it readily appears that the cytophilic pads 22 are formed on the surface of the substrate 11 and the neuronal clusters 18 are then grown on the cytophilic pads. Preferably, the cell growth is controlled so that the clusters expand over the whole surface of their respective pads.

The cytophilic pads are thus islands of material that retain cells and promote their growth, i.e. material adapted for hosting and culturing cells deposited thereon. Any appropriate material may be used to form the cytophilic pads. In particular a variety of proteins may be used, and for example, but not exclusively, Poly-D-Lysine or Poly-L-Lysine and laminin. It may be noted that the cytophilic pads 22 are generally made of material having appropriate electrical conductivity, since they are at the interface between the clusters and the electrode 20. Electrical conductivity of the pad material is however not critical, since, as is known in the art, MEA electrodes may also perform an indirect, capacitive detection, hence operating like antennas, which detect changes in electrical potential due to changes in electrical field caused by varying current flows in the culture medium.

By forming on the substrate pads of cytophilic material with desired shape and surface the selective growth of the cells on the pad surface is promoted. As a consequence by combining selective adhesion of the cells with appropriate parameters of the operating protocol the cells clusters of desired size and distribution will be obtained. Although many other parameters of the operating procedure can influence the cluster formation, by standardizing and strictly controlling them, the cell cluster size and distribution will depend only on the applied pattern.

In other words, it is possible by proper control of the operating procedure to ensure that the clusters will grow homogenously and occupy the whole area of their respective pads. Therefore, it may be noted that the areas indicated 12, 14, 16 and 18 and representing the clusters in Fig.3 can also be considered to represent simply the cytophilic pads on which the clusters are then grown/cultured, since they occupy *in fine* the same surface area.

It shall be noted that the techniques, materials and protocols required to manufacture the present biosensor are known by those skilled in the art and will not be discussed in detail herein. In particular, a variety of substrates may be used that comprise a solid layer of biocompatible material with embedded electrodes and connection circuitry; although MEAs in general are preferred.

MEA chips are conventionally made of glass and a biocompatible insulator (silicon oxide or nitride) that hosts room for the metal electrode (the electrode surface is made from a noble metal e.g. Platinum). Between the insulator and the glass layers, there are the connecting lanes that go from the electrodes to the connectors for the control system.

MEA preparation and cell seeding techniques are performed according to well-established laboratory procedures as well as the coating. Here in particular cytophilic material, i.e. adhesion promoting factors that retain and favor the growth of neural cells, are known in the art. An example of standard coating procedure is to prepare a first layer of laminin followed by a second layer of poly-L or poly-D lysine.

Furthermore, various surface engineering methods are known in the art to provide the desired surface structure for the present sensor. In particular, neuropatterning techniques like micro-contact printing, micro-spotting, inkjet printing, or carbon nano-tubes deposition may be employed to form the cytophilic pads on the substrate surface.

### Example.

For the sake of exemplification, we shall now give a detailed protocol for the preparation of the present biosensor. The substrate is a conventional MEA chip (glass substrate and Si₃N₄ insulator; Pt electrodes and gold connections).

The seeding and culture on this MEA chip where carried out as follows.

### A. MEA chip coating and sterilization

A1. Sterilization of chips wrapped in aluminium foil in a dry oven at +122°C for 2 h.
   All the following procedures are carried out under the laminar hood with sterile solutions.
A2. Placing the MEA chips in sterile Petri dishes.
A3. Transferring the pattern of adhesion-promoting agent by using PDMS stamps inked with Laminin (Sigma L2020), working solution 0.02 mg/ml.
A4. Incubation at +37°C for 3 hours.
A5. Removal of Laminin by aspiration with a sterile pipette tip connected to the pump.
A6. Coating the chips with Poly-D-lysine (PDL) (Sigma P6407) 0.01% (w/v) solution by using PDMS stamps from A3. This step is carried out with care in order to properly align the stamp and obtain the same stamp position against the chip surface as in step A3.
A7. Overnight incubation at 37°C.
A8. Washing 3 times with sterile water and drying under the laminar hood before plating the cells.

Points 3 and 6 play a role in the cell adhesion process and thus in network morphology. Other protocols may of course be followed, e.g. the use of PolyEthylenlmine (PEI) (Soussou et al. IEEE TBE 2007 vol. 54) has been suggested instead of PDL for a stronger cell to substrate adhesion.

### B. Cell preparation, thawing and plating

In this example the system is based on self-reassembled random neuronal networks from dissociated cortical neurons (usually from mouse or rat). The cell culture can come from freshly dissociated cells or cryopreserved cells. Neuronal networks express mature activity in a 3 week time.

The present protocol considers the use of Mouse C57 Cortex Neurons, Lonza M-Cx-300 (4.000.000 cells in a vial of 1 ml stored in liquid Nitrogen).
B1. Medium preparation using Lonza CC-4461 BulletKit according to the instructions in the product sheet.
B2. Cells are thawed quickly in water bath at +37°C and pre-warmed medium is added drop wise to reach the target concentration of viable cells (viability checked with Trypan Blue).
B3. 40µl of the cell suspension is added to the coated MEA-chips, and the cells are incubated for 2 hours at +37°C in a 5% CO₂ incubator.
B4. After the incubation, the medium is removed leaving a small amount to ensure that the cells do not dry out and 1 ml of pre-warmed medium is added.
B5. The MEA-chips are incubated until DIV 7 at +37°C in 5% CO₂.
B6. 50% of the medium is changed once a week for the first three weeks, then every 3 days until the beginning of the experiment and the MEA- chips kept in the incubator +37°C at 5% CO₂.

### C. Patterning

As mentioned in section A, microcontact printing is advantageously used herein for the formation of the cytophylic pads on the MEA substrate because it is a powerful method for chemical patterning of surfaces. As it is known in the art, a stamp can be designed with the desired pads dimension. Microstructured stamps can be fabricated by casting polydimethylsiloxane (PDMS) silicone elastomer against a silicon master. The silicon masters can be fabricated by photolithography or e-beam lithography. The PDMS stamps can be inked with a adhesion-promoting agent (Poly-D-Lysine, Poly-L-Lysine or laminin). [Ana Ruiz, Leonora Buzanska, Douglas Gilliland, Hubert Rauscher, Lucel Sirghi, Tomasz Sobanski, Marzena Zychowicz, Laura Ceriotti, Frederic Bretagnol, Sandra Coecke, Pascal Colpo, Francois Rossi, Micro-stamped surfaces for the patterned growth of neural stem cells, Biomaterials, Volume 29, Issue 36, December 2008, Pages 4766-4774, ISSN 0142-9612, DOI: 10.1016/j.biomaterials.2008.08.017.]

This technology allows obtaining high quality patterns by transferring layers of inks on the surface of a substrate through direct physical contact. The procedure is gentle enough to be used for printing compounds like extracellular matrix proteins (brain matrix or Laminin) or adhering molecules on surfaces (PDL, PLL, PEI etc).

It may however be noted that protein deposition on various surfaces in order to achieve network patterning can be carried out using different techniques from simple soft litographic techniques like microcontact printing (Martinoia et al, 1999; Morin et al 2006), ink-jet printing (Macis et al, 2007) to more complex procedures like parylene-C deposition and photo-lithographical patterning on silicon oxide (SiO2) surfaces and further activation via immersion in horse serum (Delivopoulosa et al, 2009).

### Data processing

Recordings and spike detection were performed by means of the MEA System from Multichannel Systems MCS GmbH (Germany). Any equivalent system ca be used, e.g. MED64 from AlfaMed Scientific Inc. (Japan), or MEA workstation from Plexon Inc. (USA).

## Claims

1. Biosensor substrate, said substrate (11) having a surface adapted for culturing cells, in particular neuronal cells, and comprising means for monitoring and/or stimulating the electrical activity of said cells including a micro-electrode array,
wherein said substrate has a structured surface with a predetermined pattern comprising spatially organised cytophilic pads (22) of controlled size for culturing said cells, **characterised in that** said cytophilic pads (22) exhibit a size distribution comprising a predetermined number of distinct size classes that is not less than four, each size class corresponding to a given range of cytophilic pad area; and wherein the cytophilic pad area between two next size classes is increased by a factor of four or more.

2. Biosensor substrate according to claim 1, comprising
four cytophilic pad size classes according to the following size ranges, expressed in equivalent diameters: 30-40 µm; 60-80 µm; 120-160 µm; 260-430 µm; or
four cytophilic pad size classes according to the following size ranges, expressed in equivalent diameters: 30-50 µm; 120-150 µm; 260-300 µm; 560-930 µm.

3. Biosensor substrate according to any one of the preceding claims, wherein one electrode (20) or more, preferably embedded in the substrate, is associated with each pad (22).

4. Biosensor substrate according to any one of the preceding claims, wherein said substrate comprises embedded electrodes and circuitry, said micro-electrode array being preferably of the planar type.

5. Biosensor comprising a biosensor substrate according to any one of the preceding claims and cells, preferably neural cells, arranged on said cytophilic pads; and
preferably said cells occupy essentially all of the area of their respective pads.

6. Biosensor according to claim 5, wherein said cells are grown in clusters (12, 14, 16, 18) according to said predetermined pattern of cytophilic pads.

7. Biosensor comprising cultured cells, namely neuronal cells, arranged on a substrate (11) according to a predetermined pattern, said substrate comprising means (20) for monitoring and/or stimulating the electrical activity of said neuronal cells including a microelectrode array, wherein
said substrate has a structured surface on which said cells are arranged according to said pattern in spatially organised clusters of controlled size (12, 14, 16, 18),
**characterised in that**
said clusters exhibit a size distribution comprising a predetermined number of distinct size classes that is not less than four, each size class corresponding to a given range of cluster area; and wherein the cluster area between two next size classes is increased by a factor of four or more.

8. Biosensor according to claim 7, wherein said structured surface comprises cytophilic pads (22) promoting cell growth and hosting said clusters, said cytophilic pads having different sizes adapted for the desired predetermined pattern of clusters.

9. Biosensor according to claim 7 or 8, wherein the clusters are independent.

10. Biosensor according to any one of claims 7 to 9, comprising
four cluster size classes according to the following size ranges, expressed in equivalent diameters: 30-40 µm; 60-80 µm; 120-160 µm; 260-430 µm; or
four cluster size classes according to the following size ranges, expressed in equivalent diameters: 30-50 µm; 120-150 µm; 260-300 µm; 560-930 µm.

11. Biosensor according to any one of claims 7 to 10, wherein said clusters comprise an aggregate of between 3 to 10 close cells at least.

12. A process for preparing a biosensor comprising the steps of:
providing a substrate having a surface and comprising means for monitoring and/or stimulating the electrical activity of cultured cells including a microelectrode array, the cells to be arranged on the substrate surface;
structuring the surface of said substrate to allow arrangement of cells thereon according to a predetermined pattern in spatially organised clusters of controlled size;
growing cells in clusters according to said predetermined pattern;
wherein said clusters exhibit a size distribution comprising a predetermined number of distinct size classes that is not less than four, each size class corresponding to a given range of cluster area; and
wherein the cluster area between two consecutive size classes is increased by a factor of four or more.

13. The process according to claim 12, wherein said step of structuring the surface of said substrate comprises forming cytophilic pads promoting cell growth of different sizes adapted for the desired predetermined pattern of clusters.

14. The process according to claim 12, wherein said cells are neuronal cells.

15. A method of conducting assays comprising:
- providing a biosensor substrate having a surface adapted for culturing cells and comprising means for monitoring and/or stimulating the electrical activity of said neuronal cells including a microelectrode array,
- growing cells, preferably neuronal cells, in clusters on the surface of said biosensor substrate to form a pattern of spatially organised clusters of controlled size,
wherein said clusters exhibit a size distribution comprising a predetermined number of distinct size classes that is not less than four, each size class corresponding to a given range of cluster area; and wherein the cluster area between two next size classes is increased by a factor of four or more;
- applying a sample to be tested onto said biosensor surface bearing said clusters; and
- monitoring the response of said clusters.

## Patentansprüche

1. Biosensorsubstrat, wobei das Substrat (11) eine Oberfläche aufweist, die an die Kultivierung von Zellen, insbesondere von neuronalen Zellen, angepasst ist, und Mittel zur Überwachung und/oder Stimulierung der elektrischen Aktivität der Zellen einschließlich eines Mikroelektrodenarrays umfasst,
wobei das Substrat eine strukturierte Oberfläche mit einem vorgegebenen Muster aufweist, das räumlich organisierte zytophile Pads (22) kontrollierter Größe zur Kultivierung der Zellen umfasst,
**dadurch gekennzeichnet, dass**
die zytophilen Pads (22) eine Größenverteilung aufweisen, die eine vorgegebene Anzahl verschiedener Größenklassen umfasst, die nicht kleiner als vier ist, wobei jede Größenklasse einem bestimmten Bereich einer zytophilen Padfläche entspricht; und
wobei die zytophile Padfläche zwischen zwei aufeinanderfolgenden Größenklassen um einen Faktor vier oder mehr vergrößert ist.

2. Biosensorsubstrat nach Anspruch 1, umfassend
vier Größenklassen zytophiler Pads gemäß den folgenden Größenbereichen, die in äquivalenten Durchmessern ausgedrückt sind: 30-40 µm; 60-80 µm; 120-160 µm; 260-430 µm; oder
vier Größenklassen zytophiler Pads gemäß den folgenden Größenbereichen, die in äquivalenten Durchmessern ausgedrückt sind: 30-50 µm; 120-150 µm; 260-300 µm; 560-930 µm.

3. Biosensorsubstrat nach irgendeinem der vorangehenden Ansprüche, wobei jedes Pads (22) eine Elektrode (20) oder mehr, vorzugsweise in das Substrat eingebettet, zugeordnet ist.

4. Biosensorsubstrat nach irgendeinem der vorangehenden Ansprüche, wobei das Substrat eingebettete Elektroden und eine eingebettete Schaltungsanordnung umfasst, wobei der Mikroelektrodenarray vorzugsweise die planare Bauart aufweist.

5. Biosensor umfassend ein Biosensorsubstrat nach irgendeinem der vorangehenden Ansprüche und Zellen, vorzugsweise Nervenzellen, die auf den zytophilen Pads angeordnet sind; und
wobei die Zellen vorzugsweise im Wesentlichen die gesamte Fläche der jeweiligen Pads einnehmen.

6. Biosensor nach Anspruch 5, wobei die Zellen in Clustern (12, 14, 16, 18) gemäß dem vorgegebenen Muster zytophiler Pads wachsen gelassen werden.

7. Biosensor umfassend kultivierte Zellen, nämlich neuronale Zellen, die auf einem Substrat (11) gemäß einem vorgegebenen Muster angeordnet sind, wobei das Substrat Mittel (20) zur Überwachung und/oder Stimulierung der elektrischen Aktivität der neuronalen Zellen einschließlich eines Mikroelektrodenarrays umfasst,
wobei das Substrat eine strukturierte Oberfläche aufweist, auf welcher die Zellen gemäß dem Muster in räumlich organisierten Clustern (12, 14, 16, 18) kontrollierter Größe angeordnet sind,
**dadurch gekennzeichnet, dass**
die Cluster eine Größenverteilung aufweisen, die eine vorgegebene Anzahl verschiedener Größenklassen umfasst, die nicht kleiner als vier ist, wobei jede Größenklasse einem bestimmten Bereich einer Clusterfläche entspricht; und
wobei die Clusterfläche zwischen zwei aufeinanderfolgenden Größenklassen um einen Faktor vier oder mehr vergrößert ist.

8. Biosensor nach Anspruch 7, wobei die strukturierte Oberfläche zytophile Pads (22) umfasst, die das Zellwachstum fördern und Wirt für die Cluster sind, wobei die zytophilen Pads verschiedene Größen aufweisen, die an das gewünschte vorgegebene Muster von Clustern angepasst sind.

9. Biosensor nach Anspruch 7 oder 8, wobei die Cluster unabhängig voneinander sind.

10. Biosensor nach irgendeinem der Ansprüche 7 bis 9, umfassend vier Clustergrößenklassen gemäß den folgenden Größenbereichen, die in äquivalenten Durchmessern ausgedrückt sind: 30-40 µm; 60-80 µm; 120-160 µm; 260-430 µm; oder
vier Clustergrößenklassen gemäß den folgenden Größenbereichen, die in äquivalenten Durchmessern ausgedrückt sind: 30-50 µm; 120-150 µm; 260-300 µm; 560-930 µm.

11. Biosensor nach irgendeinem der Ansprüche 7 bis 10, wobei die Cluster ein Aggregat mit mindestens 3 bis 10 eng benachbarten Zellen umfassen.

12. Verfahren zur Herstellung eines Biosensors, umfassend die folgenden Schritte:
Bereitstellen eines Substrats, das eine Oberfläche aufweist und Mittel zur Überwachung und/oder Stimulierung der elektrischen Aktivität kultivierter Zellen einschließlich eines Mikroelektrodenarrays umfasst, wobei die Zellen auf der Substratoberfläche anzuordnen sind;
Strukturieren der Oberfläche des Substrats, um die Anordnung von Zellen darauf gemäß einem vorgegebenen Muster in räumlich organisierten Clustern kontrollierter Größe zu ermöglichen;
Wachsenlassen von Zellen in Clustern gemäß dem vorgegebenen Muster;
wobei die Cluster eine Größenverteilung aufweisen, die eine vorgegebene Anzahl verschiedener Größenklassen umfasst, die nicht kleiner als vier ist, wobei jede Größenklasse einem bestimmten Bereich einer Clusterfläche entspricht; und
wobei die Clusterfläche zwischen zwei aufeinanderfolgenden Größenklassen um einen Faktor vier oder mehr vergrößert ist.

13. Verfahren nach Anspruch 12, wobei der Schritt zum Strukturieren der Oberfläche des Substrats das Bilden zytophiler Pads umfasst, die das Zellwachstum verschiedener Größen fördern, die an das gewünschte vorgegebene Muster von Clustern angepasst sind.

14. Verfahren nach Anspruch 12, wobei die Zellen neuronale Zellen sind.

15. Verfahren zur Durchführung von Assays, umfassend:
- Bereitstellen eines Biosensorsubstrats, das eine Oberfläche aufweist, die an die Kultivierung von Zellen angepasst ist, und Mittel zur Überwachung und/oder Stimulierung der elektrischen Aktivität der neuronalen Zellen einschließlich eines Mikroelektrodenarrays umfasst,
- Wachsenlassen von Zellen, vorzugsweise von neuronalen Zellen, in Clustern auf der Oberfläche des Biosensorsubstrats, um ein Muster von räumlich organisierten Clustern kontrollierter Größe zu bilden,
wobei die Cluster eine Größenverteilung aufweisen, die eine vorgegebene Anzahl verschiedener Größenklassen umfasst, die nicht kleiner als vier ist, wobei jede Größenklasse einem bestimmten Bereich einer Clusterfläche entspricht; und wobei die Clusterfläche zwischen zwei aufeinanderfolgenden Größenklassen um einen Faktor vier oder mehr vergrößert ist;
- Aufbringen einer zu prüfenden Probe auf die die Cluster tragende Biosensoroberfläche; und
- Überwachen des Ansprechverhaltens der Cluster.

## Revendications

1. Substrat de biocapteur, ledit substrat (11) ayant une surface adaptée pour cultiver des cellules, en particulier des cellules neuronales, et comprenant des moyens pour surveiller et/ou stimuler l'activité électrique desdites cellules comprenant un réseau de microélectrodes,
dans lequel ledit substrat a une surface structurée avec un motif prédéterminé comprenant des blocs cytophiles organisés dans l'espace (22) de taille contrôlée pour cultiver lesdites cellules, **caractérisé en ce que** lesdits blocs cytophiles (22) présentent une répartition par taille comprenant un nombre prédéterminé de classes de taille distinctes qui n'est pas inférieur à quatre, et chaque classe de taille correspondant à une plage donnée d'aire de bloc cytophile ; et
dans lequel l'aire de bloc cytophile entre deux classes de taille consécutives est augmentée d'un facteur de quatre ou plus.

2. Substrat de biocapteur selon la revendication 1, comprenant
quatre classes de taille de blocs cytophiles selon les plages de taille suivantes, exprimées en diamètres équivalents : 30-40 µm ; 60-80 µm ; 120-160 µm ; 260-430µm ; ou
quatre classes de taille de blocs cytophiles selon les plages de taille suivantes, exprimées en diamètres équivalents: 30-50 µm ; 120-150 µm ; 260-300 µm ; 560-930 µm.

3. Substrat de biocapteur selon l'une quelconque des revendications précédentes, dans lequel une électrode (20) ou plus, de préférence incorporée dans le substrat, est associée à chaque bloc (22).

4. Substrat de biocapteur selon l'une quelconque des revendications précédentes, dans lequel ledit substrat comprend des électrodes incorporées et un circuit, ledit réseau de microélectrodes étant de préférence du type planaire.

5. Biocapteur comprenant un substrat de biocapteur selon l'une quelconque des revendications précédentes et des cellules, de préférence des cellules neuronales, agencées sur lesdits blocs cytophiles ; et
de préférence lesdites cellules occupent essentiellement la totalité de l'aire de leurs blocs respectifs.

6. Biocapteur selon la revendication 5, dans lequel lesdites cellules sont développées en agrégats (12, 14, 16, 18) selon ledit motif prédéterminé de blocs cytophiles.

7. Biocapteur comprenant des cellules cultivées, à savoir des cellules neuronales, agencées sur un substrat (11) selon un motif prédéterminé, ledit substrat comprenant des moyens (20) pour surveiller et/ou stimuler l'activité électrique desdites cellules neuronales comprenant un réseau de microélectrodes,
dans lequel ledit substrat a une surface structurée sur laquelle lesdites cellules sont agencées selon ledit motif dans des agrégats de taille contrôlée organisés dans l'espace (12, 14, 16, 18),
**caractérisé en ce que** lesdits agrégats présentent une répartition par taille comprenant un nombre prédéterminé de classes de taille distinctes qui n'est pas inférieur à quatre, chaque classe de taille correspondant à une plage donnée d'aire d'agrégat ; et
dans lequel l'aire d'agrégat entre deux classes de taille consécutives est augmentée d'un facteur de quatre ou plus.

8. Biocapteur selon la revendication 7, dans lequel ladite surface structurée comprend des blocs cytophiles (22) facilitant la croissance de cellules et hébergeant lesdits agrégats, lesdits blocs cytophiles ayant différentes tailles adaptées pour le motif prédéterminé souhaité d'agrégats.

9. Biocapteur selon la revendication 7 ou 8, dans lequel les agrégats sont indépendants.

10. Biocapteur selon l'une quelconque des revendications 7 à 9, comprenant quatre classes de taille d'agrégat selon les plages de taille suivantes, exprimées en diamètres équivalents: 30-40 µm ; 60-80 µm ; 120-160 µm ; 260-430µm ; ou
quatre classes de taille de blocs cytophiles selon les plages de taille suivantes, exprimées en diamètres équivalents: 30-50 µm ; 120-150 µm ; 260-300 µm ; 560-930 µm.

11. Biocapteur selon l'une quelconque des revendications 7 à 10, dans lequel lesdits agrégats comprennent un agrégat d'au moins 3 à 10 cellules proches.

12. Procédé de préparation d'un biocapteur comprenant les étapes consistant à :
fournir un substrat ayant une surface et comprenant des moyens pour surveiller et/ou stimuler l'activité électrique de cellules cultivées comprenant un réseau de microélectrodes, les cellules devant être disposées sur la surface de substrat ;
structurer la surface dudit substrat pour permettre une disposition des cellules dessus selon un motif prédéterminé dans des agrégats de taille contrôlée organisés dans l'espace ;
développer les cellules dans des agrégats selon ledit motif prédéterminé ;
dans lequel lesdits agrégats présentent une répartition par taille comprenant un nombre prédéterminé de classes de taille distinctes qui n'est pas inférieur à quatre, chaque classe de taille correspondant à une plage donnée d'aire d'agrégat ; et
dans lequel l'aire d'agrégat entre deux classes de taille consécutives est augmentée d'un facteur de quatre ou plus.

13. Procédé selon la revendication 12, dans lequel ladite étape consistant à structurer la surface dudit substrat comprend la formation de blocs cytophiles facilitant la croissance de cellules de tailles différentes adaptées au motif prédéterminé souhaité d'agrégats.

14. Procédé selon la revendication 12, dans lequel lesdites cellules sont des cellules neuronales.

15. Procédé de conduite d'essais comprenant :
- la fourniture d'un substrat de biocapteur ayant une surface adaptée pour cultiver des cellules et comprenant des moyens pour surveiller et/ou stimuler l'activité électrique desdites cellules neuronales comprenant un réseau de microélectrodes,
- le développement de cellules, de préférence des cellules neuronales, en agrégats sur la surface dudit substrat de biocapteur pour former un motif d'agrégats de taille contrôlée organisés dans l'espace,
dans lequel lesdits agrégats présentent une répartition par taille comprenant un nombre prédéterminé de classes de taille distinctes qui n'est pas inférieur à quatre, chaque classe de taille correspondant à une plage donnée d'aire d'agrégat ; et dans lequel l'aire d'agrégat entre deux classes de taille consécutives est augmentée d'un facteur de quatre ou plus ;
- l'application d'un échantillon à tester sur ladite surface de biocapteur porteuse desdits agrégats : et
- la surveillance de la réponse desdits agrégats.
